# EUROPEAN PATENT SPECIFICATION

(11) **EP 3 445 974 B1**
(45) Date of publication and mention of the grant of the patent: **18.11.2026**
(21) Application number: 17786597.9
(22) Date of filing: 20.04.2017
(51) Int. Cl.: F04D 29/66, A61F 7/00, F04D 29/58, F04D 29/70

(54) **AIR WARMER**
LUFTWÄRMER
RÉCHAUFFEUR D'AIR

(30) Priority: 21.04.2016 US 201662325502 P; 19.04.2017 US 201715491004
(43) Date of publication of application: 27.02.2019
(73) Proprietor: ICU Medical, Inc., San Clemente, CA 92673 (US)
(72) Inventor: PETERSON, Benjamin, James, Apple Valley MN 55124 (US); REDA, Ahmed, Brooklyn Center MN (US); SANBORN, Jonathan, Harold, St Louis Park MN 55416 (US); TREPPA, Michael, A., Blaine MN 55434 (US)
(74) Representative: Schlich
(86) International application number: PCT/US2017/028479
(87) International publication number: WO 2017/184797

(56) References cited:
- CN-U- 2 073 503
- CN-U- 2 073 503
- CN-U- 204 512 005
- US-A- 5 567 127
- US-A- 6 143 020
- US-A- 6 143 020
- US-A1- 2004 005 225
- US-A1- 2008 127 976
- US-A1- 2011 200 426
- US-A1- 2015 176 860
- US-A1- 2015 176 860

## Description

### Field of the Invention

The present invention relates to an air blower and particularly an air convective warmer that is adapted to provide an optimum throughput of temperature regulated air to inflate a convective blanket while operating at a reduced noise level.

### Background of the Invention

An air convective warmer is used to inflate a convective blanket that provides a constant stream of heated air to warm a patient to regulate the body temperature of the patient. There are myriad air convective warmers being sold in the market, including an air convective warmer sold by the assignee under the trade name EQUATOR^{®}. Most of these air convective warmers tend to produce a high level of noise. The noise is mostly due to the air turbulence that results from the rotation of the fan or impeller in the air plenum of the warmer needed to suck air into the plenum from the environment and to propel a stream of pressurized air, heated or otherwise, to inflate the convective blanket.

Attempts have been made to reduce the noise of air convective warmers. One such attempt, as disclosed in US 6,126,393 and its related US 6,254,337, relies on the relative orientation of the air inlet and air outlet of the warmer. Another attempt, as disclosed in US 5,733,320, uses noise cancellation components. Yet other attempts to reduce the noise level of an air convective warmer are disclosed in US 7,037,068 and US 8,720,220, both assigned to the assignee of the instant application. The '068 patent discloses forming indentations at the interior surface of the plenum chamber, and the '220 patent discloses the use of a noise reduction air filter. US 2015/176860 discloses an air warmer which has a housing whereinto is positioned a noise reduction mechanism having a casing that encases a sound reducing structure having a maze-like cavity. CN2073503 discloses a noise-eliminating device for air-blowers which is provided with a casing, an inlet casing fixedly connected with the casing, sound absorbing material and a micro perforated plate. US2011/0200426 discloses a noise reduction device for a blower includes a noise reduction member. The noise reduction member has a noise reduction chamber and a noise reduction opening defined therein, so that a part of air flowing into, through or out of the blower can enter the noise reduction chamber via the noise reduction opening. US6143020 discloses a fluid blanket warming system for a patient which includes a thermal blanket unit for receiving heated gas through a coupling port. US2008/127976 and CN204512005 refer to further documents relevant for the present invention.

### Summary of the Present Invention

The inventive air blower of the present invention is defined by appended claim 1. Preferred embodiments are disclosed in the dependent claims. The air blower is preferably an air convective warmer which has an air blower that preferably has a motor assembly that includes a fan or impeller positioned in an air plenum. The impeller is preferably connected to a shaft that extends from a motor. The plenum has an air inlet and an air outlet, and preferably has a cavity so that air can circulate within the plenum. Positioned to the inlet side of the plenum is a first noise reduction structure, or an inlet muffler, that has two cylindrical sections and a through passage longitudinally extending along the sections to effect a fluid conduit connecting the inlet and an outlet of the inlet muffler. One of the cylindrical sections is designed to attenuate high frequency noise while the other of the sections is designed to attenuate low frequency noise. The section that attenuates the lower frequency noise has a larger diameter than the section that reduces the high frequency noise. Thus, the inlet muffler for the exemplar air blower has a telescopic shape, with the wider section (in terms of its diameter) being positioned adjacent to the inlet side of the plenum, and the outlet of the inlet muffler in alignment with the inlet of the plenum which establishes a fluid communication path between the inlet muffler and the air plenum of the motor assembly.

A filter is preferably fittingly coupled to the inlet side of the air inlet muffler to filter the air sucked into the convective warmer from the environment.

The outlet of the air plenum is preferably in fluid communication with an inlet of a heater plenum. The heater plenum is preferably configured to have an elbow shape to reduce the turbulence of air flowing through it. A plurality of heating elements is preferably mounted in the heater plenum to heat the air flow that enters it from the air plenum.

The outlet of the heater plenum is preferably in fluid communication with an inlet of an outlet noise reduction structure, or an outlet muffler, that also has an outlet. The outlet muffler preferably has two sections. One of the sections is preferably designed to reduce high frequency noise and the other of the sections preferably is designed to attenuate low frequency noise. A central through passage preferably extends longitudinally along the two sections to effect a fluid conduit connecting the inlet and outlet of the outlet muffler. The heated air from the heater plenum flows through the fluid conduit of the outlet muffler. At the outlet of the outlet muffler there is preferably an extension adapted to be connected to a machine end of an air hose which other end is connected to an input port of a convective blanket.

When the air convective blower is in operation, air from the environment is sucked into the air plenum and a stream of pressurized airflow having a certain velocity is output from the air plenum. The air output from the air plenum is preferably routed to the heater plenum where it is heated. The heated air then preferably passes through the outlet muffler to inflate the convective blanket. With an inlet muffler and an outlet muffler preferably sandwiching the air plenum of the motor assembly, the noise resulting from the operation of the air convective blower when the impeller is in motion is substantially reduced. Lab tests show that the inventive convective air warmer has a noise level that is below most, if not all, of the known convective air warmers.

Each section of each of the inlet and outlet mufflers may preferably be fitted with a noise absorbent material such as a sound absorbing foam. Alternatively, one of the sections, for example the lower frequency noise attenuation section, is preferably fitted with the noise absorbent material while the high frequency noise attenuation section is preferably configured as a Helmholtz chamber. The inlet muffler may preferably be a one piece integral component having a telescopic shape with a large cylindrical section and a small cylindrical section. The outlet muffler may also preferably be a one piece integral component but with an oblong large section and a small cylindrical section. The various components of the inventive air convective warmer may preferably be encased in a housing that has displays, inputs and other interfacing means to enable a user or clinician to operate the air convective warmer.

The present disclosure is also directed to an air blower that comprises a motor assembly including a motor, a plenum and an impeller preferably workingly coupled to the motor preferably movably positioned inside the plenum; the plenum having an inlet and an outlet; an inlet muffler preferably coupled to the plenum, the inlet muffler including a housing preferably having an outer wall and an inlet through passage connecting an inlet and an outlet of the inlet muffler, the outlet of the inlet through passage in fluid communication with the inlet of the plenum, a sound absorbent material preferably fitted between the outer wall of the housing and an inner circumferential wall of the housing that defines the inlet through passage; preferably an outlet muffler including a housing having an outer wall and an outlet through passage connecting an inlet and an outlet of the outlet muffler, the outlet muffler positioned relative to the plenum so that the outlet through passage is in fluid communication with the outlet of the plenum, a sound absorbent material preferably fitted between the outer wall and an inner circumferential wall of the housing that defines the outlet through passage; and preferably an air filter coupled to the inlet muffler so that air sucked into the plenum is filtered by the air filter; wherein when the motor assembly is in operation, the air sucked into the inlet through passage is passed to the plenum and output therefrom to the outlet through passage as a stream of air flow; and wherein sound resulting at least from air turbulence due to movement of the air in the plenum during operation of the motor assembly is reduced by both the inlet and outlet mufflers.

The instant invention is moreover directed to an air blower that comprises: a motor assembly including a motor, a plenum and an impeller preferably workingly coupled to the motor movably positioned inside the plenum, the plenum having an inlet and an outlet; an inlet muffler including an inlet through passage having an outlet in fluid communication with the inlet of the plenum, a low frequency noise attenuation section and a high frequency attenuation section; preferably an outlet muffler including an outlet through passage having an inlet in fluid communication with the outlet of the plenum, a low frequency noise attenuation section and a high frequency attenuation section; wherein when the motor is activated to rotate the impeller in the plenum, air is sucked into the inlet muffler and a stream of air flow is output from the outlet of the outlet muffler; and wherein the inlet and outlet mufflers act to reduce noise resulting from operation of the air blower.

### Brief Description of the Figures

The present invention will become apparent and the invention itself will be best understood with reference to the following description of the invention taken in conjunction with the accompanying drawings, wherein:
Fig. 1 is a perspective view of an embodiment of the inventive air convective warmer;
Fig. 2 is another perspective view of the inventive air convective warmer;
Fig. 3 is another view of the air convective warmer with the motor assembly removed from the frame to show the inlet opening to the heater plenum;
Fig. 4 is yet another perspective view of the air convective warmer where the housing of the heater plenum has been removed to show the heater elements;
Fig. 5 shows cut away views of the inlet and outlet mufflers of the air convective warmer;
Fig. 6 is a disassembled illustration of the inventive air convective warmer showing its various components;
Fig. 7 shows the disassembled components of the inventive air convective warmer at a different perspective ;
Fig. 8 is a semi-disassembled view of the air convective warmer showing the different major components thereof and the different airflow paths therethrough;
Figs. 9A, 9B and 9C are exemplar housings to encase the inventive air convective warmer;
Fig. 10 shows a fully assembled inventive air convective warmer on a stand;
Fig. 11 is an illustration of the system architecture of the inventive air convective warmer and its use;
Figs. 12A and 12B are NC (noise criteria) and NR (noise rating) charts showing the noise level generated by the inventive air convective warmer; and
Fig. 13 is an illustration showing an exemplar positioning of the sensors at the hose and the outlet of the air convective warmer that may be used to provide a built-in sensor reference circuitry to enable different types of air hoses to be used with the air convective warmer without the need to re-calibrate the warmer for each type of the air hoses.

### Detailed Description of the Invention

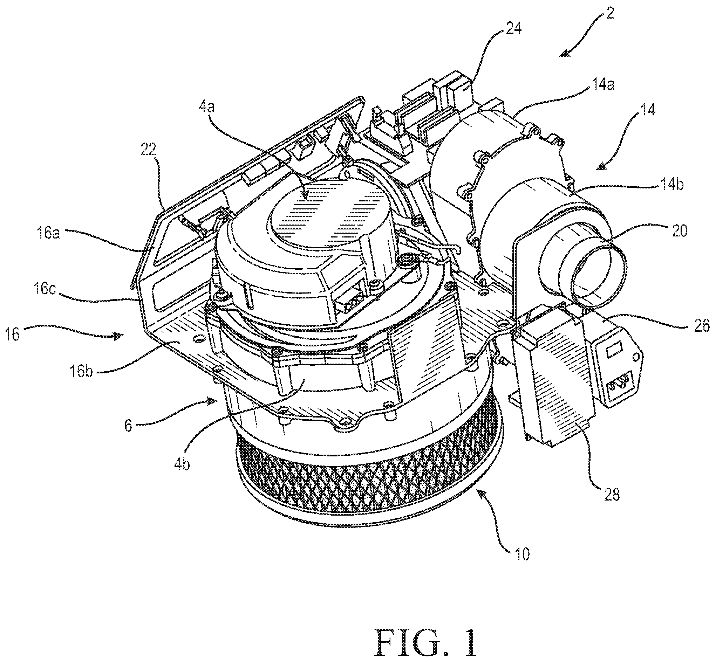

With reference to Figs. 1-6, an embodiment of the inventive air convective warmer 2 is shown to have a motor assembly 4, a first noise reduction structure referenced hereinbelow as an inlet muffler 6, a filter 10, a heater plenum 12 and an outlet noise reduction structure referred to hereinbelow as an outlet muffler 14. All of the major components of the embodiment air convective warmer 2 are either attached, or positioned relative, to a structural frame 16.

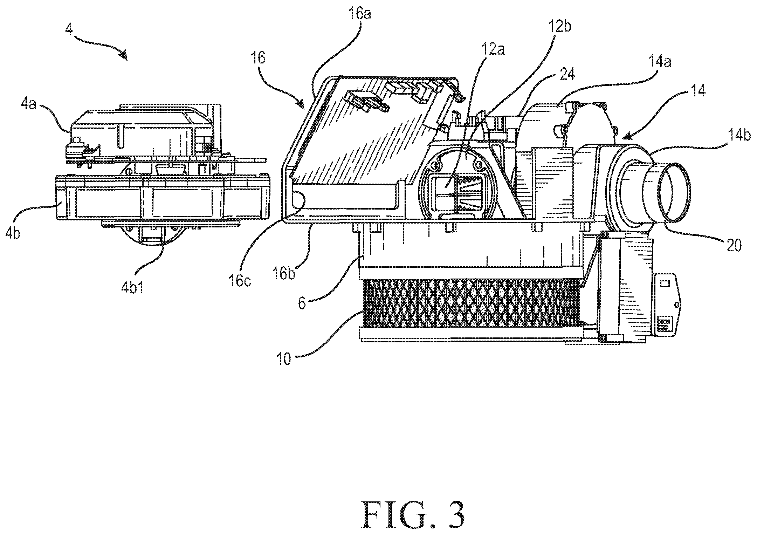

As best shown in Figs. 3, 6 and 7, the motor assembly has a motor 4a and an impeller, or a fan, that is housed in an air plenum 4b. As is well known, the impeller is workingly coupled to motor 4a by means of a drive shaft (not shown) that extends from the motor. Thus, when the motor is energized, the impeller is rotated in air plenum 4b. The impeller for the motor assembly is a centrifugal fan and the motor assembly may be purchased from ebm-papst Landshut GmbH of Germany as Product No. RG148/1200-3612.

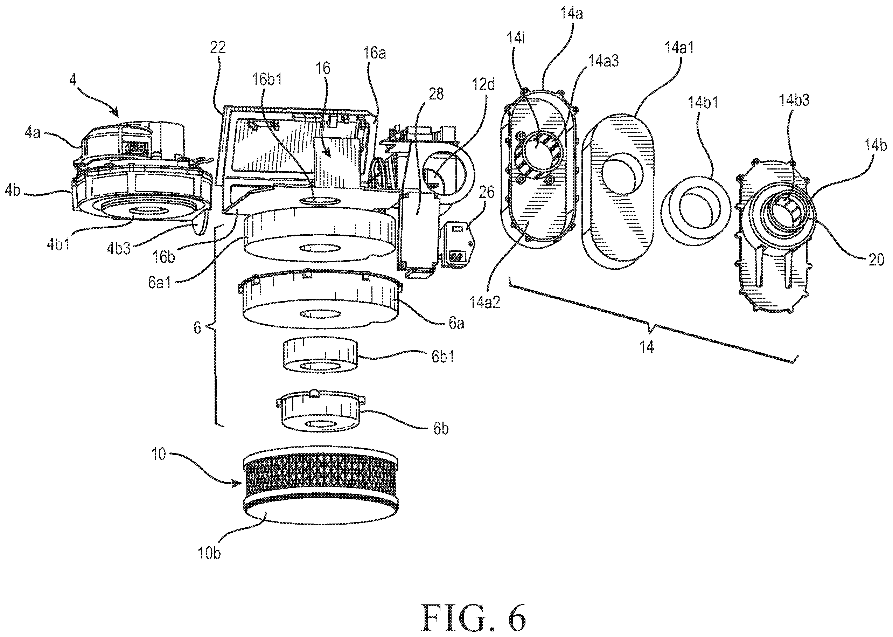

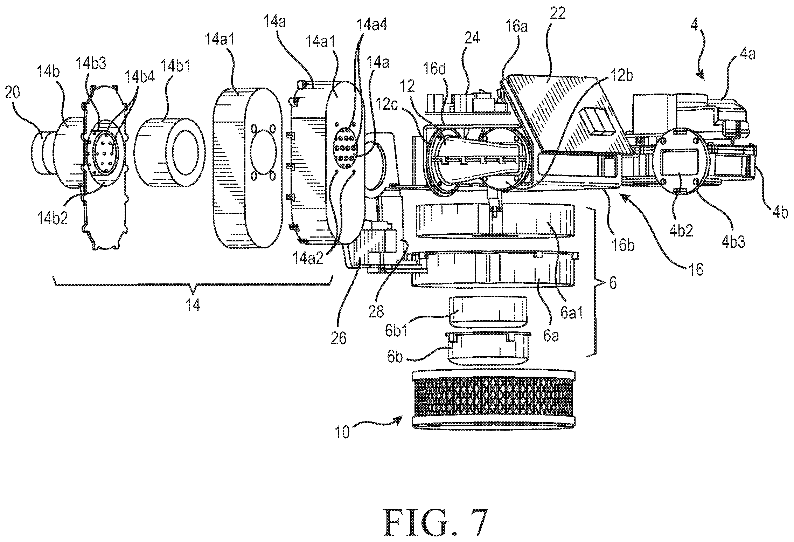

Air plenum 4b has an inlet and an outlet. Inlet 4b1 is best shown in Fig. 6. Outlet 4b2 (Fig. 7) is at the side of plenum 4b that faces inlet opening 12a of heater plenum 12, as per shown in Fig. 3. When mounted to base portion 16a of frame 16, the aperture opening at inlet 4b1 of air plenum 4b is aligned with opening 16b1 of frame 16 (Fig. 6). Thus, when motor 4a is energized, the impeller inside plenum 4b is rotated so that air is sucked into the cavity of plenum 4b via inlet 4b1 and a stream of pressurized airflow is output from outlet 4b2 of air plenum 4b.

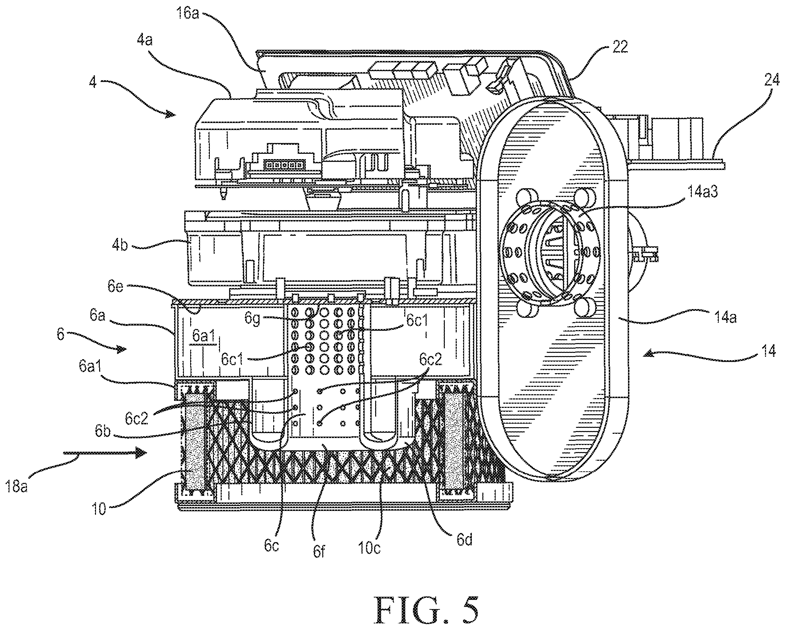

As shown in the figures, attached to the underside of base 16b of frame structure 16 is inlet muffler 6. As best shown in Figs. 5-8, the exemplar embodiment inlet muffler 6 has two cylindrical sections 6a and 6b. For explanation purpose, sections 6a and 6b are shown to be separate elements in Figs. 6 and 7, albeit sections 6a and 6b in actuality may be formed as sections of a one piece integral structure such as the inlet muffler 6 shown in Fig. 5. In any event, cylindrical section 6a is a wider section than section 6b and thus section 6a has a larger diameter than section 6b. The respective diameters and configurations of sections 6a and 6b are designed to enable those sections to attenuate or reduce noises of different frequency ranges. Larger diameter section 6a is designed to attenuate the lower frequency noises while smaller diameter section 6b is designed to attenuate noises of higher frequencies across the broadband of noise frequencies. As best shown in Fig. 5, sections 6a and 6b have a common central coaxial bore 6c that effects a through passage longitudinally along inlet muffler 6 between its lower end 6d and upper end 6e, where inlet 6f and outlet 6g, respectively, of inlet muffler 6 are located. Thus, a fluid communication path is established between air plenum 4b and the environment via the through passage as inlet 6f of inlet muffler 6 is positioned inside of and adapted to receive air filtered by filter 10. Given that outlet 6g is in alignment with inlet 4b1, when the motor assembly is in operation, the rotation of the impeller within plenum 4b creates a vacuum therein to suck filtered air from the environment into the air plenum 4b by way of inlet muffler 6.

The air that passes through the inlet muffler 6 into plenum 4b is filtered by filter 10. As shown in Fig. 5, filter 10 is removably coupled to the outer circumferential periphery of the underside of section 6a by a gasket 6a1, so that section 6b extends within cavity 10c of filter 10. Given that base 10b of filter 10 is air impermeable for the exemplar embodiment, the air being sucked into air plenum 4b through inlet muffler 6 flows into cavity 10c through the filter material of filter 10 in a sideways direction as indicated by directional arrow 18a.

To aid in the reduction of noise, section 6a and 6b of inlet muffler 6 each are fitted with a noise absorbent material 6a1 and 6b1, respectively, for the exemplar embodiment air blower as shown. The noise absorbent material may be a noise reduction foam purchased from the 3M company, or from the Acoustical Solutions company of Richmond, VA under the trade name AlphaComposite^{®} acoustic foam. The foam may be formed as a pad that substantially form fits within each of the respective spaces defined between the outer walls of the sections and the inner circumferential wall of inlet muffler 6 that defines the common coaxial bore that forms through passage 6c

To further enhance the reduction of noise, a plurality of apertures or holes are formed or provided at the inner circumferential or tubular wall of inlet muffler 6 that forms passage 6c. The size of the holes 6c1 at the inner circumferential wall that defines passage 6c at section 6a has a larger diameter than the diameter of the holes 6c2 at the circumferential wall that defines passage 6c at section 6b. The holes extend through the inner circumferential wall to noise absorbent foams 6a1 and 6b1 so that passage 6c is exposed to the noise absorbent foam. As a result, a portion of the noise resulting from the moving air traversing along passage 6c may be absorbed by the foam.

With the configuration of the inlet muffler 6 as shown, section 6a acts as a filter to attenuate the noises of lower frequencies that result from the turbulence of the air flow created by the movement of the impeller as the sucked in air moves through the air plenum. The noises of higher frequencies are attenuated by filter section 6b of inlet muffler 6. It should be appreciated that the various dimensions of the inlet muffler are dependent on the motor assembly, specifically the size of air plenum 4b and the rotational speed of the impeller that is required to generate the amount of airflow necessary to inflate a convective blanket connected to the outlet extension of the air convective warmer, as will be discussed below.

Although the inlet muffler 6 as discussed above has its high frequency attenuation section 6b fitted with a noise absorbent foam material, another embodiment of the inlet muffler requires the noise absorbent foam material be fitted to only one of the muffler sections, for example section 6a to attenuate the low frequency noise. For the high frequency noise attenuation, instead of fitted with a foam pad, section 6b may be configured as a Helmholtz chamber so that the resonance within the chamber acts to attenuate the high frequency noise. As is known, a Helmholtz chamber may be designed in accordance to the following.

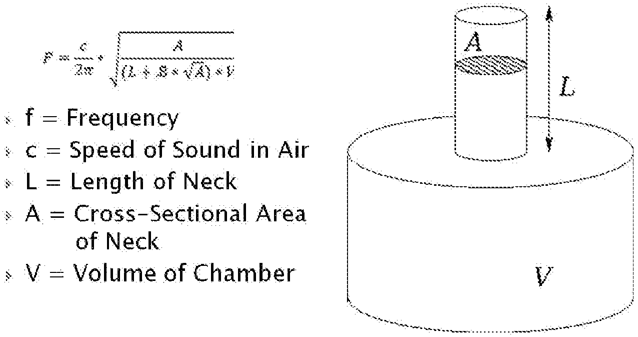

For the inventive air blower, the inlet and outlet mufflers each may have a section configured as a Helmholtz chamber in the manner as illustrated above so that only the low noise frequency attenuation section of the inlet and outlet mufflers 6 is fitted with a noise absorbent foam material.

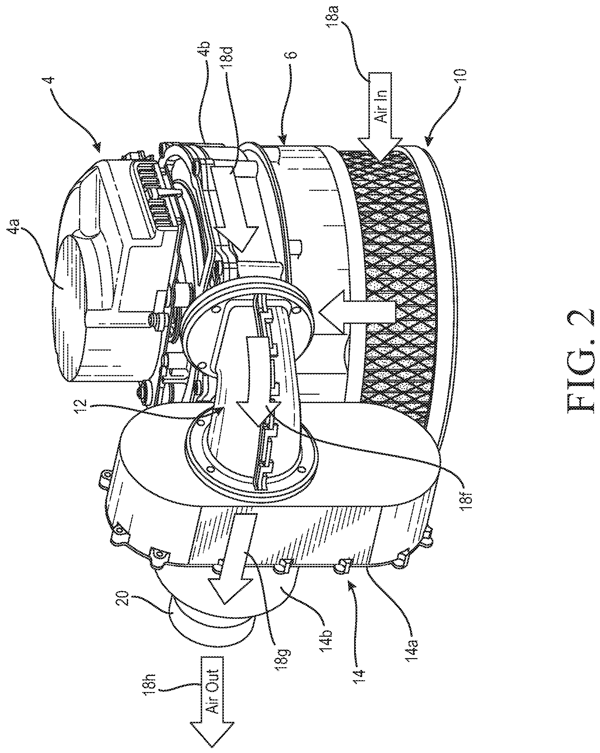

As best shown in Figs. 2, 3 and 7, outlet 4b2 of air plenum 4b is coupled to inlet 12a of heater plenum 12 by means of an outlet flange 4b3 at plenum 4b and an inlet flange 12b of heater plenum 12. When coupled, outlet 4b2 is in alignment with inlet 12a to effect an airflow path, i.e., a fluid communication path, between air plenum 4b and heater plenum 12.

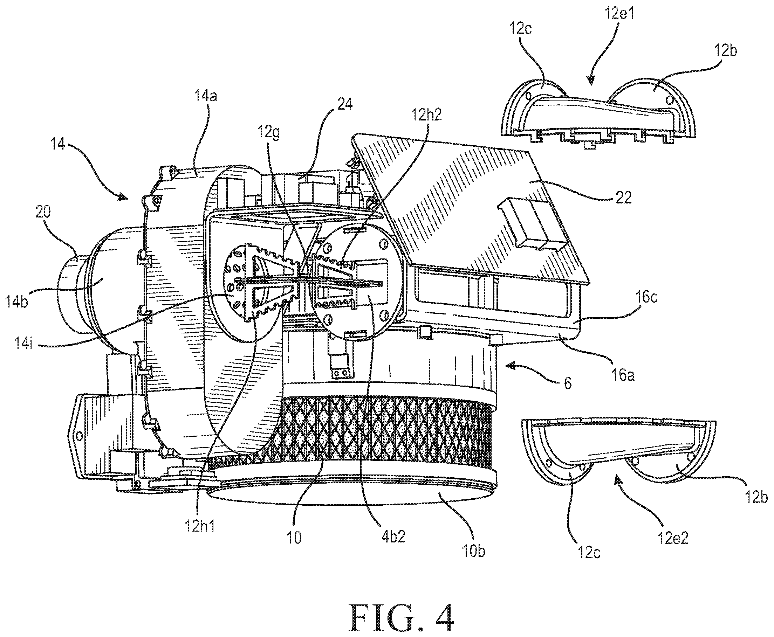

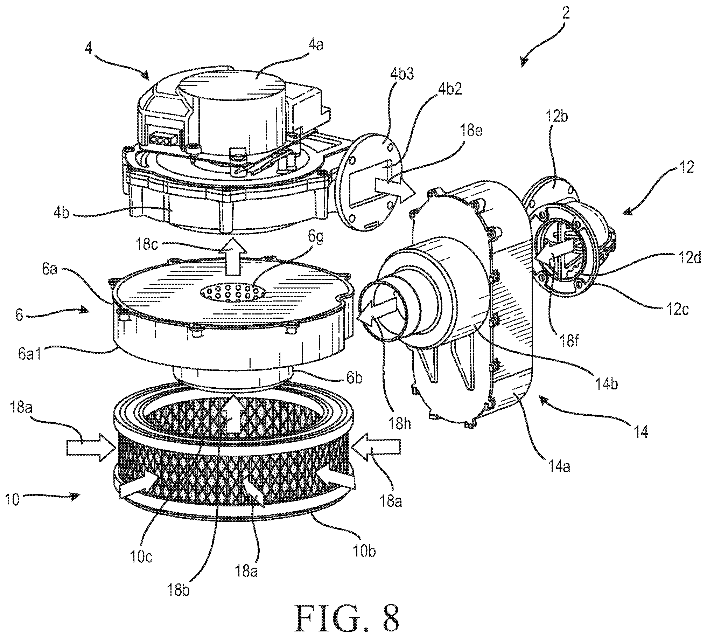

Although heater plenum 12 shown in the figures is in the shape of an elbow for the exemplar embodiment air convective warmer, it should be appreciated that the heater plenum 12 may have other shapes. Heater plenum 12 is configured in the form of an elbow for the exemplar embodiment air convective warmer to effect a compact enclosure and to smooth out the turbulence of the airflow exiting plenum 4b. Provided inside heater plenum 12 are heater elements. In Fig. 4, the upper and lower portions 12e1 and 12e2 of the heater plenum are removed to one side of the figure to expose the heater elements, which may be heater coils 12h1 and 12h2 mounted to a support 12g. The air output from outlet 4b2 of air plenum 4b is heated by the heater elements in heater plenum 12 before exiting from outlet 12d (Fig. 8).

With reference to Figs. 2 and 8, the output air is referenced by directional arrow 18d. The direction of the airflow from filter 10 into inlet muffler 6 is indicated by directional arrow 18b. The airflow from the outlet 6g of inlet muffler 6 to the inlet of air plenum 4b is indicated by directional arrow 18c. The movement of the airflow in air plenum 4b is indicated by directional airflow 18d. And the stream of airflow output from outlet 4b2 of air plenum 4b is designated by directional arrow 18e. It is this stream of pressurized airflow that is fed to heater plenum 12 as discussed above.

Outlet 12d of heater plenum 12 is in fluid communication with inlet 14i of the oblong section 14a of outlet muffler 14. As best shown in Fig. 7, an outlet flange 12e of heater plenum 12 is connected to an inlet surface 14a1 of oblong section 14a of outlet muffler 14 in a conventional known manner, as for example by rivets or screws. Alignment holes 14a2 are provided on inlet surface 14a1 so that inlet 14i of outlet muffler 14 may be correctly aligned with outlet 12d of heater plenum 12.

Outlet muffler 14 has a large oblong section 14a and a small cylindrical section 14b. Although shown to have an oblong structure, section 14a may be of other shapes including cylindrical. As shown in Figs. 6 and 7, a noise absorbent foam pad 14a1 is fitted between tubular wall 14a3 and the outer wall of oblong section 14a , and a noise absorbent foam ring 14b2 is fitted between tubular wall 14b3 and the outer wall of cylindrical section 14b. Tubular walls 14a3 and 14b3 define the through passage for outlet muffler 14.

Same as the inlet muffler, there are holes along both of tubular walls 14a3 and 14b3 to expose the through passage to the noise absorbent foam to enhance noise reduction for outlet muffler 14. The holes 14a4 at tubular wall 14a3 have a larger diameter than the diameter of holes 14b4 at tubular wall 14b3. Similar to inlet muffler 6, larger section 14a attenuates the noise of the lower frequencies while smaller section 14b attenuates the noise of the higher frequencies of the air stream that passes through outlet muffler 14. Similar to inlet muffler 6, section 14b of outlet muffler 14 may be replaced by a Helmholtz chamber to attenuate the high frequency noises from the output airflow stream.

Although sections 14a and 14b of the outlet muffler 14 are shown as separate elements, in practice, both of those sections may be formed as a one piece integral component with the noise absorbent foam fitted therein, or a one piece integral component that includes a Helmholtz chamber. Both of the inlet and outlet mufflers may be molded as a one piece plastic component.

Integrated to the outlet side of section 14b of outlet muffler 14 is an outlet extension or coupler 20 adapted to connect to one end of an air hose, not shown, which other end is connected to a convective blanket, as is conventionally known, so that the convective blanket may be inflated by the stream of air output from the air convective warmer. Although not shown, outlet extension 20 may extend outside the housing that encloses the air convective warmer shown in Figs. 1-8 for easy connection to the air hose. Outlet extension 20 may simply be referred to as the air outlet of the convective warmer that outputs the temperature regulated air, for example the air heated when it passes through the heater plenum 12. The stream of output air is referenced by directional arrow 18h shown in Figs. 2 and 8.

A logic circuit board 22 mounted to frame section 16a of frame 16 provides the control to power the motor assembly as well as to energize the heating elements 12h1 and 12h2 for the convective air warmer. Circuit board 22 includes all of the low voltage electronics and microprocessor controllers, as well as the software needed to operate the air convective warmer. The software and electronics are well known and may be borrowed from the electronics that are used in the above-noted EQUATOR^{®} air warmer. The circuit board also provides the electronics and circuits for a user or clinician to select the temperature for the output air. Sensors may be provided at the outlet extension 20 and/or the air hose to provide a feedback to maintain a regulated temperature of the air output from the air convective warmer. There is also a power circuit board 24 mounted to frame portion 16d. Power circuit board 24 has all of the power transistors, heat sinks and the power supply for providing the required voltage to control board 22, as well as the AC power that is needed to energize the heater elements 12h1 and 12h2 and motor 4a. The power to the heater elements is regulated by power circuit board 24 to control the temperature of the airflow being heated. A conventional power entry module 26 provides power to circuit board 24 from an electrical outlet. Further provided is a conventional regulated 24 volt power supply module 28.

With the inlet and outlet of the air plenum 4b of the motor assembly in fluid communication with the inlet muffler 6 and the outlet muffler 14, respectively, noises of both high and low frequencies are attenuated or reduced when air is output from the warmer to inflate a convective blanket at an optimum flow rate, for example at 0.0179 cubic metres/second (38 cfm (cubic feet per minute)). The outlet muffler also minimizes the turbulence and interference that is inherent in the stream of air flow from the blower, as well as the pressure drop of the output air stream. The noise level of the embodiment of the inventive air blower during operation is measured to be less than 43 dBA (sound pressure level), which is a reduction in noise from the above-noted EQUATOR^{®} convective warmer.

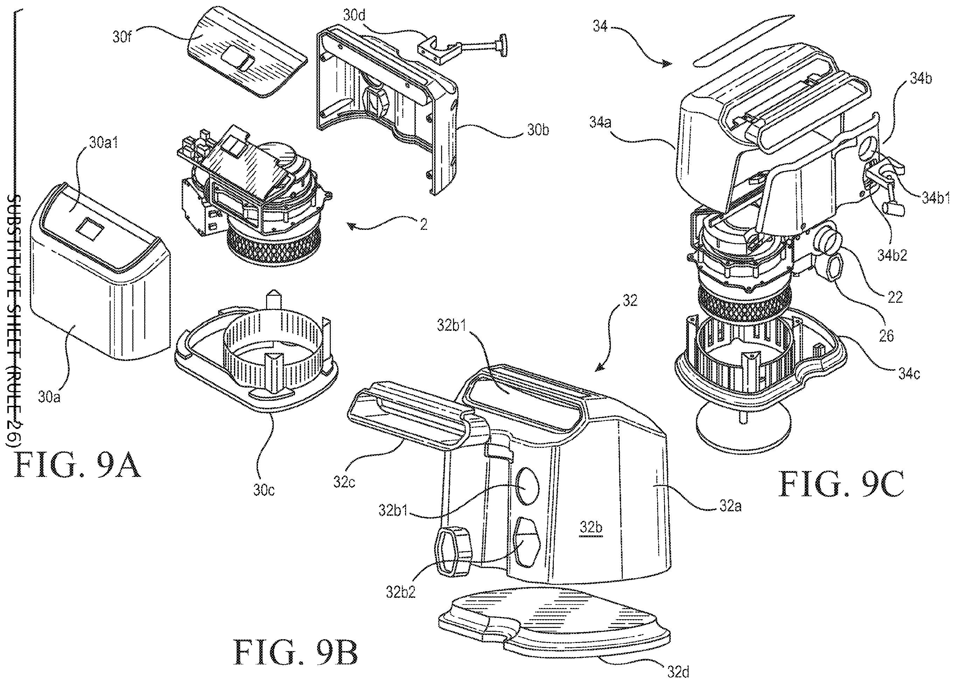

Figs. 9A-9C are exemplar housings that may be used to enclose air convective warmer 2. The housing in Fig. 9A is made up of a front half casing 30a and a back half casing 30b as well as a base 30c onto which filter 10 is placed. To the back of casing 30b there may be added a mechanism 30d to enable the housing to be held onto a pole of a stand, not shown. A faceplate 30f that may be placed onto the viewing surface 30a1 at the front half casing 30a to provide the display and interface means for the user to view and interact with the convective warmer, for example setting the required temperature and alarm limits as is well known. Fig. 9B shows another exemplar housing 32 that may be used to house the inventive air convective warmer. Housing 32 also has a front casing 32a, a back casing 32b, a rear insert 32c to fit over an opening 32b1 of the casing as well as a base 32d. Fig. 9C shows yet another exemplar housing 34 having at its front casing 34a and back casing 34b superposed over the air convective warmer, as well as a base 34c for supporting the air convective warmer. Openings 34b1 and 34b2 in the back casing 34b are for outlet extension 22 and power entry module 26. The same types of openings are shown as 32b1 and 32b2 in the Fig. 9B embodiment. Similar openings are shown in the back casing 30b of the Fig. 9A embodiment housing. The respective peripheries for each of the bases 30c, 32d and 34c curve inwardly in an upward manner toward the platform of the bases where the air filter is positioned. In the exemplar embodiments of Figs. 9A and 9C, the filter is positioned within a circumferential picket fence cage integrally extending upwardly from the base. The curved shape of the periphery of each of the bases provides a smoother inlet to the air plenum and therefore contributes to the attenuation or reduction of the noise of the air being sucked into the filter and then into the air plenum. As a result, the nosie level of the air intake at the air inlet for the air warmer housed in the exemplar housings is low. This low noise air intake in turn contributes in keeping the noise level of the inventive air warmer low. An additional advantage of the base having an upwardly and inwardly curved periphery is that it keeps away water ingress to the housing of the air warmer.

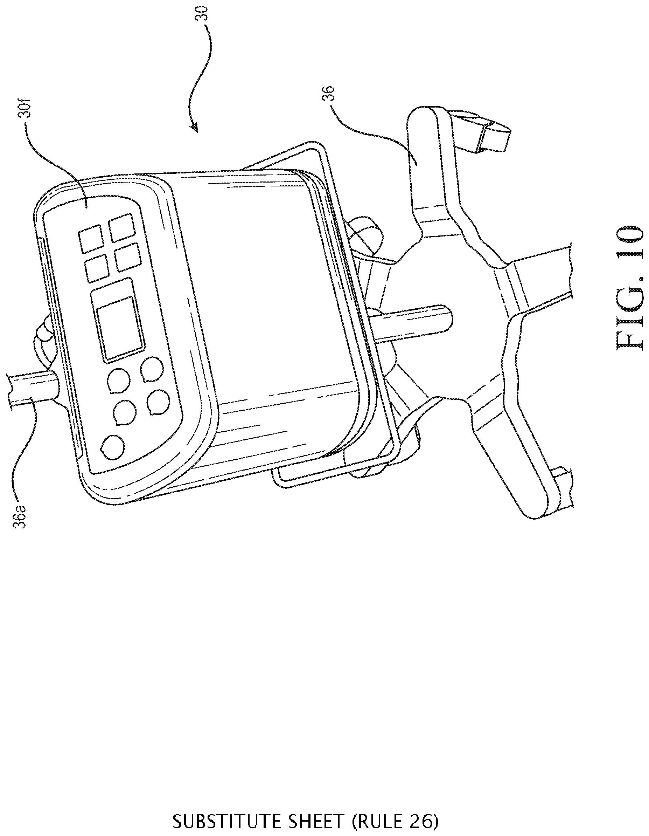

Fig. 10 shows the inventive air convective warmer housed in the exemplar Fig. 9A housing mounted to a pole 36a of a stand 36. For the exemplar housing of Fig. 10, face 30f at the front casing shows the temperature of output air, the various inlet and outlet temperature set, as well as the power switch and other displays that assist a clinician in the use of the inventive air convective warmer.

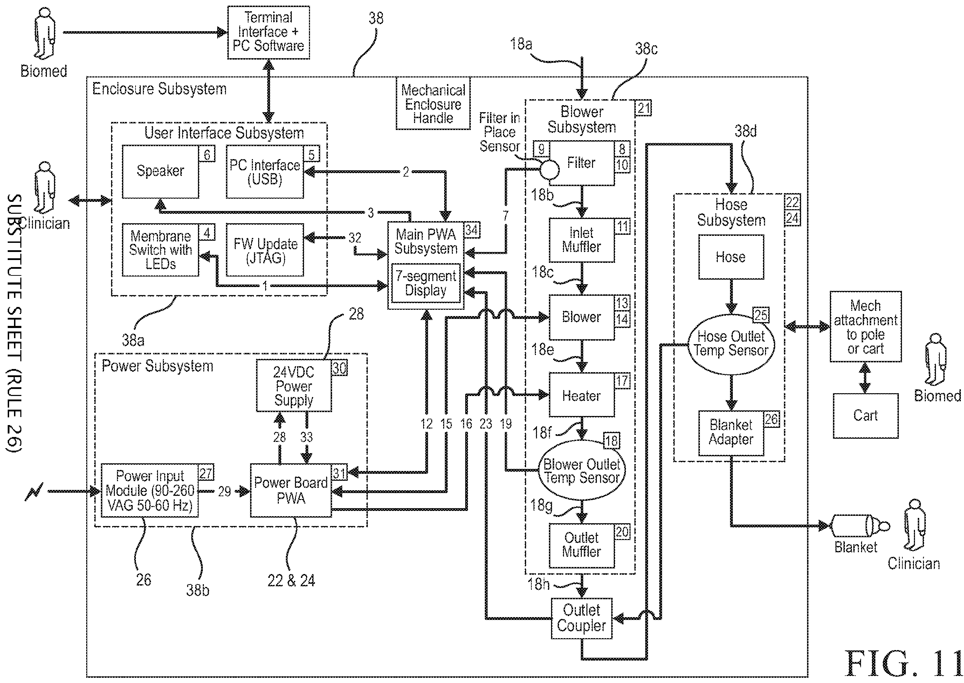

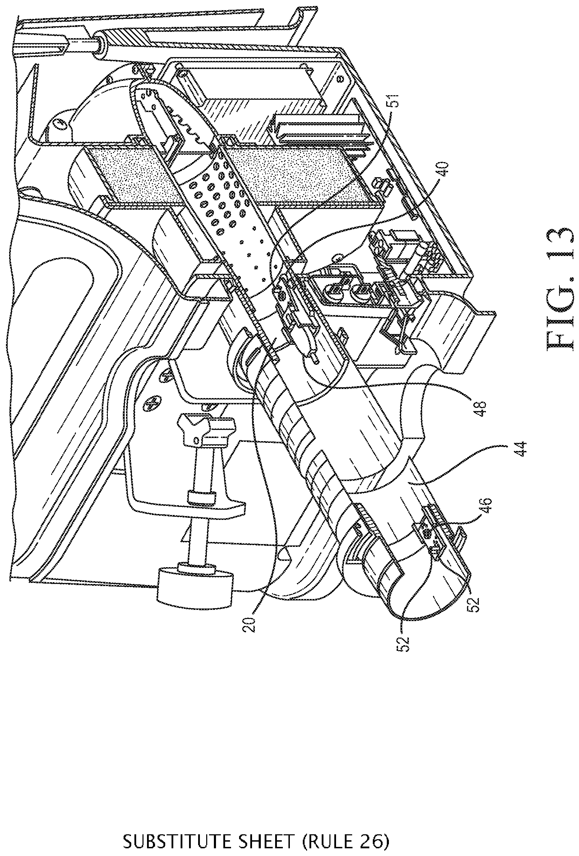

Fig. 11 is a system architecture drawing that shows an overall view of the inventive air convective warmer *vis-a-vis* the convective blanket. As shown, the housing is identified as the enclosure subsystem with the user interface subsystem and the power subsystem in dotted line boxes. The enclosure subsystem 38 may be equated to the exemplar housing 30 shown in Fig. 10 with the user interface subsystem 38a corresponding to the faceplate 30f. The power subsystem 38b is a combination of the power input module 26, the power supply 28, the circuit board 22 and the power board 24. The filter 10, the inlet muffler 6, the motor assembly (designated as the blower), the heater plenum (designated as the heater), and the outlet blower are shown in the blower subsystem 38c. The air flow, referenced by directional arrows 18a-18h, are also shown in the blower subsystem 38c. The stream of air output from the convective warmer is sent to the hose subsystem 38d, which includes the air hose, to connect to the outlet extension 20. In essence, the hose subsystem 38d is an air hose that may include sensors for providing feedback to the controller at circuit board 22 to control the temperature of the airflow being output to inflate the blanket. The sensors may be used as part of a built-in reference circuit to allow different types of air hoses to be coupled to the air warmer without the need to re-calibrate the air warmer. The built-in reference circuit will be discussed below with reference to Fig. 13.

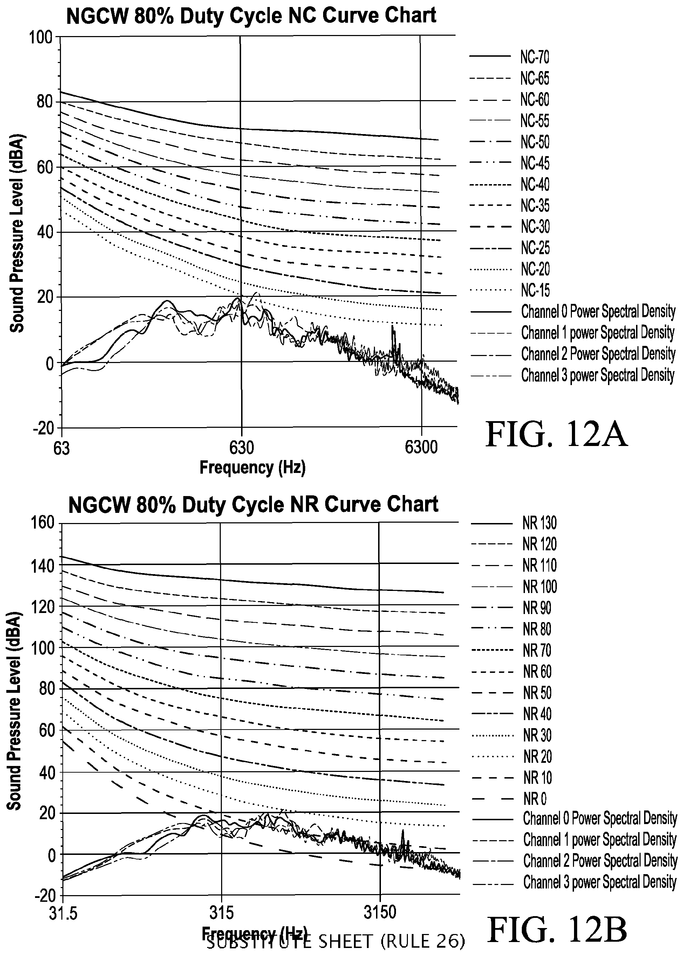

Figs. 12A and 12B are charts illustrating the sound quality of the inventive warm air blower. These charts describe the measured noise level having taken into account the frequency content of the noise. Fig. 12A is a NC (Noise Criteria) curve chart while Fig. 12B is a NR (Noise Rating) curve chart each showing the noise level generated by the inventive air blower operating at 80% duty cycle. The Fig. 12A NC chart was first developed in the late 1950's and is commonly used in the United States. The Fig. 12B NR chart is based on the ANSI Standard S 12.2-1995 and is commonly used in Europe. As can be seen, the sound level dBA *vis-a-vis* the frequencies resulting from the operation of the air warmer of the instant invention at a duty cycle of 80% is approximately at or below 20 dBA for both the NC and NR charts. It has been determined that the inventive blower operating at 80% duty cycle is fully capable of inflating most, if not all, of conventional convective blankets including those sold by the assignee of the instant application.

The level of noise, or simply noise, of the air blower of the instant invention, which may technically be referred to as the Leq (Equivalent continuous sound level) Average Sound Levels (or simply dBA), was measured under different sets of conditions whereby components were added as well as removed from the overall air blower. For example, different levels of noise resulting from the operation of the air blower were measured with the inlet muffler and/or the outlet muffler removed, only one of the mufflers removed, and the shapes of the respective portions of each of the inlet and outlet mufflers reconfigured. Also, different types of noise insulating or reduction materials (or no material) were fitted, form fitted or otherwise, within each of the inlet and outlet mufflers, either separately or together, to determine the amount of noise reduced by the use of those materials. Some of the sound insulating foam materials used include Basotect G+ from the BASF company, polyurethane 2 lb, polyurethane 4 lb, 3M Thinsulate, as well as air (no material). The same foam material may be used in both of the inlet and outlet mufflers, or different foam materials may be separately used in the mufflers.

By varying the configuration of the mufflers as well as fitting the mufflers with the different types of sound reduction material discussed above, the level of noise, i.e., dBA, resulting from the operation of the air blower of the instant invention based on the configuration of the mufflers as discussed above is determined to be confined to between approximately 44.5 dBA (air with no sound reduction material) to approximately 41 dBA (with Basotect G+). With all components added to the air blower including the air filter and the heater fixedly positioned within the elbow of the air blower as described above, the level of noise when the inventive air blower is in operation is measured to be between approximately 42 dBA and 43 dBA. A noise level of approximately 42.6 dBA was measured over time with the inventive air blower in continuous operation.

As discussed above, sensors are provided at the outlet extension 20 as well as the air hose to provide a feedback to maintain a regulated temperature of the air output from the air convective warmer. For the instant invention, there is also provided a built-in sensor (or thermistor) reference circuit that allows the clinician to switch to different types of air hoses, for example switching from an air hose used to inflate an adult blanket to an air hose used to inflate a pediatric blanket, without having to re-calibrate the air warmer to take into account the different dimensions of the blankets, as well as the different air hoses used to establish a fluid flow path between the blankets and the air convective warmer. The built-in reference circuit utilizes the sensors provided at the patient end of the air hose and the sensor provided at the outlet of the convective warmer. One such configuration of the placement of the sensors is described in US 6,143,020, assigned to the assignee of the instant application. In the '020 patent, two sensors are mounted to a cross structure attached to the patient end of the air hose to provide a temperature feedback to the air warmer. The sensors are conventional temperature sensing devices such as thermocouples, thermistor or resistive temperature devices (RTD), semi-conductor diode-junction or integrated circuit temperature sensors.

Fig. 13 is an illustration of an alternate exemplar sensor configuration for providing a feedback to control the temperature of the air output from the air convective warmer. As shown, a circuit board 40 supporting a sensor S1 is mounted at the outlet extension 20 of the air convective warmer. Mounted to the patient end of air hose 44 is another circuit board 46 that has mounted thereto two sensors S2 and S2'. Each of sensors S2 and S2' operates to measure the temperature of the heated air output from the convective warmer. Having two sensors provides redundancy to the system. As further shown, a connector 48 connected to the circuit board 40 represents the electrical connection between the circuit boards 40 and 46 that enables the temperature at the end of the air hose measured by sensors S2 and S2' to be fed to circuit board 40 as a remote temperature signal. As should be appreciated, the temperature at the patient end of the air hose corresponds to the temperature of the air being input to inflate the blanket. By way of circuit board 40, signals representing the air temperature measured at the patient end of the air hose and the temperature of the heated air measured at the outlet of the air blower by sensor S1 are sent to the control system 38 of the air warmer. Based on the difference of the temperatures at the outlet of the warmer and at the patient end of the hose, a feedback is provided to the control system 38 to maintain regulate a desired temperature for the air output from the air convective warmer.

In addition to providing a feedback, the inventive air convective warmer has a built-in reference circuit that enables the warmer to use the aforenoted sensors to automatically sense the air hose that is connected to the warmer, for example whether it is an air hose used for inflating an adult underbody blanket or an air hose used to inflate a pediatric blanket. The circuit utilizes the sensors to establish an output table by applying a linear calibration to the existing values of the sensors with reference to the different types of air hoses that may be used with the air convective warmer for inflating the different convective blankets.

One example of utilizing the values of the different sensors (or thermistors) is by using a resistor ladder scheme, for example a R-2R network to establish a number of outputs that respectively correspond to the different types of air hoses. Thus, when a given air hose is coupled to the air warmer, an output that corresponds to the characteristics of the given air hose is provided to the controller of the air warmer to automatically adjust the rate and amount of heated air to output from the air warmer, i.e., to effect a substantially optimal air output for the given air hose. The respective characteristics of the different air hoses may be measured empirically. An exemplar R-2R network circuit adapted to provide eight outputs is shown hereinbelow. Eight outputs are believed to be adequate to accommodate the different air hoses that are adapted to be used with the inventive air convective warmer. However, if there are additional types of air hoses and thus additional outputs are required, additional resistance series may be added to the circuit to provide outputs of 10, 12, 14, etc.

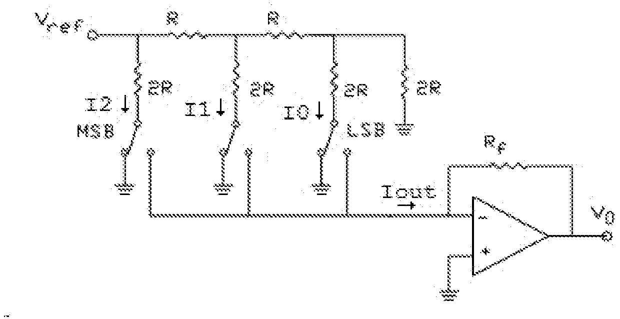

The above exemplar R-2R network circuit can be seen to be like a current source whose output depends on switch setting B2, B1, B0 (MSB to LSB) that controls I2, I1, I0 respectively. ${I}_{out} = {V}_{ref} \times 8 R \left(4B2+2B1+B0\right)$ (1)

Including the op amp which behaves like I-V converter, the voltage output can be obtained per the following equation. ${V}_{out} = - {V}_{ref} \times {R}_{f} \times 8 R \left(4B2+2B1+B0\right)$ (2) Letting V_{ref} = 1 and R_{f} = 2R, the following output table is obtained.

**Output Table**

| B2 | B1 | B0 | Digital Value | Vₒᵤₜ |
|---|---|---|---|---|
| 0 | 0 | 0 | 0 | 0 |
| 0 | 0 | 1 | 1 | -0.25 |
| 0 | 1 | 0 | 2 | -0.5 |
| 0 | 1 | 1 | 3 | -0.75 |
| 1 | 0 | 0 | 4 | -1.0 |
| 1 | 0 | 1 | 5 | -1.25 |
| 1 | 1 | 0 | 6 | -1.51 |
| 1 | 1 | 1 | 7 | -1.75 |

As the R-2R network circuit is well known and equations (1) and (2) are likewise well known, it suffices to note that the above R-2R circuit acts to provide different values, in this instance eight, that are adapted to correspond to the different characteristics of the different air hoses. For example, value "1" having the exemplar output voltage of -0.25 may be construed to correspond to the coupling to the inventive air warmer of an air hose optimally adapted to be used to inflate an adult blanket, for example the adult underbody blanket SWU-2119 sold by the assignee of the instant application. Value "2", which corresponds to an exemplar output voltage of -0.5, may be construed to mean that an air hose coupled to the inventive air convective warmer is to be used to inflate an underbody pediatric blanket, for example the pediatric underbody blanket SW-2009 sold by the assignee of the instant application. This correspondence of the value outputs from the above R-2R circuit therefore provides a built-in table for the controller of the air convective warmer to automatically and optimally adjust its air output to accommodate the air hose connected to the warmer. For example, when an air hose for inflating the exemplar adult SWU-2119 blanket is connected to the inventive air warmer, the controller of the air warmer would effect the blower to output air at a highest rate of about 11.36 m/s (2236 ft/min) which is approximately 0.0254 cubic metres/second (approximately 52 CFM (Cubic Feet per Minute)). For the exemplar pediatric SW-2009 blanket, the controller of the air warmer would effect the blower to output the heated air at a lowest rate of about 10.29m/s (2026 ft/min) which is approximately 0.0222 cubic metres/second (approximately 47 CFM). The rate of the air output from the air warmer may be manually adjusted by the clinician if needed.

For the exemplar R-2R circuit, 15°C may be used as a first reference temperature and 44°C may be used as the second reference temperature to compensate for the thermistor values, so that the output voltages in practice may be different from those shown in the table. With the built-in reference circuit, a clinician can switch air hoses to connect different convective blankets to the inventive air convective warmer without having to re-calibrate the air output from the air warmer. Another advantage of the built-in reference circuit is that it also removes the need to have any annual or bi-annual calibration of the air warmer, as the air output is calibrated automatically in accordance with the values of the output table. Yet another advantage is that the built-in circuit does not require additional components, insofar as it utilizes the existing sensors.

Inasmuch as the present invention is subject to variations, modification and changes in detail, it is intended that all matters described throughout this specification and shown in the accompanying drawings be interpreted as illustrative only and not in a limiting sense. For example, instead of the oblong configuration for the large section of the outlet muffler, that section of the outlet muffler may be configured in a cylindrical configuration to reduce air turbulence. So, too, instead of fitting a noise absorbent foam in at least one of the sections in each of the mufflers, with the proper design, both sections of the inlet and outlet mufflers may be configured as Helmholtz chambers. Alternatively, a different type of noise absorbent material other than foam may be used to enhance the reduction of noise during the operation of the warmer.

## Claims

1. An air blower (2) having a plenum (4b) wherein an impeller is movably positioned, the plenum having an inlet (4b1) and an outlet (4b2), a motor (4a) working cooperatively with the impeller for rotating the impeller and an inlet noise reduction structure (6) positioned relative to the plenum (4b) including a housing having at least one wide section (6a) and one narrow section (6b), the wide section (6a) having a larger diameter than the narrow section (6b), the wide section (6a) is designed to attenuate the lower frequency noises while narrow section (6b) is designed to attenuate noises of higher frequencies across the broadband of noise frequencies, the wide and narrow sections (6a, 6b) encircling a common central coaxial bore (6c) defined by a tubular wall that extends through the wide and narrow sections to effect a through passage (6c) having an inlet (6f) open to the environment, the one wide section (6a) positioned adjacent to the plenum (4b) so that an outlet (6g) of the inlet through passage (6c) is in fluid communication with the inlet (4b1) of the plenum (4b) to establish a fluid communication path between the inlet through passage (6c) and the plenum (4b), a noise absorbent material (6a1, 6b1) fitted to at least one of the wide and narrow sections (6a, 6b) to surround a corresponding portion of the inlet through passage (6c); wherein when the motor (4a) is activated, the impeller is rotated to draw air into the plenum (4b) from the inlet through passage (6c) and to output a stream of air flow through the outlet (4b2) of the plenum; and wherein a portion of the noise resulting from the movement of air in the plenum (4b) is reduced by the inlet noise reduction structure (6).

2. The air blower of claim 1, further comprising:
an outlet noise reduction structure (14) including a housing having at least a large section (14a) and a small section (14b) encircling an outlet through passage (14a3, 14b3), the large section (14a) positioned relative to the plenum (4b) so that a fluid communication path is established between an inlet (14i) of the outlet noise reduction structure (14), the outlet through passage (14a3, 14b3) and the plenum (4b), a noise absorbent material (14a1, 14b1) fitted to at least one of the large and small sections (14a, 14b) to surround a corresponding portion of the outlet through passage (14a3, 14b3);
wherein the outlet noise reduction structure (14) together with the inlet noise reduction structure (6) substantially reduce the noise resulting from the movement of air through the plenum (4b).

3. The air blower of claim 2, wherein a plurality of holes (6c1) formed along the tubular wall, the holes at the tubular wall along the wide section (6a) having a larger dimension than the holes at the tubular wall along the narrow section (6b);
wherein the outlet through passage (14a3, 14b3) is defined by an internal circumferential wall that extends along the large and small sections (14a1, 14b1), a plurality of holes formed along the circumferential wall, the holes at the circumferential wall along the large section having a larger dimension than the holes at the circumferential wall along the small section.

4. The air blower of claim 2, wherein each of the wide and narrow sections (6a, 6b) of the inlet noise reduction structure (6) is fitted with the noise absorbent material (6a1, 6b1); and
wherein each of the large and small sections (14a, 14b) of the outlet noise reduction structure (14) is fitted with the noise reduction material (14a1, 14b1);
whereby the noise resulting from the operation of the blower is measured to be in a range of approximately 41 dBA to 44.5 dBA.

5. The air blower of claim 1, further comprising at least one heater element (12h1, 12h2) positioned relative to the outlet (4b2) of the plenum (4b) to heat the air flow output from the outlet of the plenum.

6. The air blower of claim 2, further comprising a heater plenum (12) wherein at least one heater element (12h1, 12h2) is positioned, the heater plenum (12) interposed between the plenum (4b) and the outlet noise reduction structure (14), the heater plenum (12) having an inlet (12a) to receive the air output from the plenum (4b) and an outlet (12d) to output the air having been heated by the heater element (12h1, 12h2) to the inlet (14i) of the outlet noise reduction structure (14) so that heated air is output from the outlet (20) of the outlet through passage (14a3, 14b3) and
wherein the outlet noise reduction structure (14) includes an outlet extension (20) adapted to connect to one end of an air hose (44) which other end is connectable to a convective blanket so that the blanket may be inflated by the heated air output from the outlet noise reduction structure (14).

7. The air blower of claim 1, wherein the wide section (6a) is defined by a wide outer circumferential wall and the narrow section (6b) is defined by a narrow outer circumferential wall that has a diameter smaller than the diameter of the wide outer circumferential wall, a circumferential flange formed where the wide section (6a) meets the narrow section (6b), the air blower further comprising:
an air filter (10) removably attached to the circumferential flange, the filter including a circumferential air passable barrier defining a cavity (10c) encircling the narrow section (6b);
wherein air is drawn into the cavity (10c) to the inlet through passage (6c) when the air blower is in operation.

8. The air blower of claim 4, wherein the outlet through passage (14a3, 14b3) is formed by an internal circumferential wall that extends along the large and small sections (14a, 14b); and
wherein the internal circumferential wall and the tubular wall of each of the inlet and outlet through passages (6c; 14a3, 14b3) is circumscribed by the noise absorbent material (6a1, 6b1; 14a1, 14b1).

9. The air blower of claim 1, wherein the wide section (6b) of the housing is fitted with the noise absorbent material (6a1, 6b1; 14a1, 14b1) and the narrow sections is a Helmholtz chamber.

10. The air blower of claim 2, wherein the outlet through passage (14a3, 14b3) is formed at least within the large and small sections (14a, 14b) of the housing of the outlet noise reduction structure (14);
wherein one of the large and small sections (14a, 14b) of the housing is fitted with the noise absorbent material (14a1, 14b1) and other of the large and small sections is a Helmholtz chamber;
wherein the noise absorbent fitted section attenuates low frequency noise and the Helmholtz chamber attenuates high frequency noise.

11. The air blower of claim 1, further comprising an air outlet (20) adapted to couple to different types of air hoses each provided at its patient end at least one sensor (s2, s2'), at least an other sensor (s1) provided at the air outlet (20) of the air blower, the one sensor (s2, s2') measuring the temperature of the heated air at the patient end of an air hose (44) coupled to the air outlet (20) and the other sensor (s1) measuring the temperature of the heated air at the air outlet, a circuit that utilizes the values of the one and other sensors with reference values to establish a reference table of stepped values that correspond to each type of the different types of air hoses adapted to be coupled to the air outlet (20), wherein when a given air hose is coupled to the air outlet, the circuit effects the motor (4a) to rotate the impeller at a rate in accordance to a step value that corresponds to the type of air hose said given air hose is.

12. The air blower of claim 1, wherein the inlet noise structure (6) comprises
an inlet muffler (6) coupled to the plenum (4b), the inlet muffler (6) including the housing (6a, 6b) having an outer wall and a wide section (6a) and a narrow section (6b), the inlet through passage (6c) in fluid communication with the inlet (4b1) of the plenum (4b), the sound absorbent material (6a1, 6b1) fitted between the outer wall of the wide section (6a) of the housing (6a, 6b) and tubular wall of the housing that defines the inlet through passage (6c); the air blower further comprising
an outlet muffler (14) including a second housing (14a, 14b) having an outer wall and an outlet through passage (14a3, 14b3), the outlet muffler (14) positioned relative to the plenum with the outlet through passage (14a3, 14b3) in fluid communication with the outlet (4b2) of the plenum, a sound absorbent material (14a1, 14b1) fitted between the outer wall and an inner circumferential wall of the second housing that defines the outlet through passage (14a3, 14b3); and
an air filter coupled to the inlet muffler to filter air sucked into the inlet through passage (6c) when the motor assembly is in operation;
wherein when the motor (4a) is in operation, the air sucked into the inlet of the inlet through passage (6c) passes to the plenum and outputs therefrom to the outlet through passage (14a3, 14b3) as a stream of air flow; and
wherein sound resulting at least from air turbulence due to movement of the air in the plenum during operation of the motor assembly is reduced by the inlet and outlet mufflers.

13. The air blower of claim 2, wherein the inlet noise reduction structure (6) is an inlet muffler including the wide section (6a) and the narrow section (6b); and
wherein the outlet noise reduction structure (14) is an outlet muffler including the large section (14a) acting as a low frequency noise attenuation section to attenuate noise of the low frequencies and the small section (14b) acting as a high frequency attenuation section to attenuate noise of the higher frequencies;
wherein when the motor is activated to rotate the impeller in the plenum, air is sucked into the inlet muffler and a stream of air flow is output from the outlet muffler; and
wherein the inlet and outlet mufflers act to reduce noise resulting from operation of the air blower.

14. The air blower of claim 13, further comprising:
an air filter (10) fitted over the inlet (6f) of the inlet muffler (6) to filter the air sucked into the inlet muffler;
a casing (32) for housing at least the motor assembly (4), the inlet and outlet mufflers (6, 14) and the heater plenum (12);
wherein the noise resulting from the operation of the air blower is measured to be between approximately 42 dBA to 43 dBA,

15. The air blower of claim 13, wherein at least one of the low frequency and high frequency attenuation sections (14a,15b) in the outlet muffler (6, 14) is fitted with a noise absorbent material (6a1, 6b1; 14a1, 14b1).

## Patentansprüche

1. Luftgebläse (2), aufweisend einen Luftraum (4b), wobei ein Laufrad beweglich positioniert ist, wobei der Luftraum einen Einlass (4b1) und einen Auslass (4b2) aufweist, einen Motor (4a), der mit dem Laufrad zum Drehen des Laufrads kooperativ zusammenarbeitet, und eine Einlassgeräuschreduktionsstruktur (6), die in Bezug auf den Luftraum (4b) positioniert ist, beinhaltend ein Gehäuse mit mindestens einem breiten Bereich (6a) und einem schmalen Bereich (6b), wobei der breite Bereich (6a) einen größeren Durchmesser als der schmale Bereich (6b) aufweist, der breite Bereich (6a) dazu konstruiert ist, die Geräusche niedrigerer Frequenz zu dämpfen, während schmaler Bereich (6b) dazu konstruiert ist, Geräusche höherer Frequenzen über das Breitband von Geräuschfrequenzen zu dämpfen, wobei der breite und der schmale Bereich (6a, 6b) eine gemeinsame zentrale koaxiale Bohrung (6c) umgeben, die durch eine rohrförmige Wand definiert ist, die sich durch den breiten und den schmalen Bereich erstreckt, um einen Durchgang (6c) mit einem Einlass (6f) herbeizuführen, der zu der Umgebung offen ist, wobei der eine breite Bereich (6a) angrenzend an den Luftraum (4b) so positioniert ist, dass ein Auslass (6g) des Einlassdurchgangs (6c) in Fluidkommunikation mit dem Einlass (4b1) des Luftraums (4b) steht, um einen Fluidkommunikationsweg zwischen dem Einlassdurchgang (6c) und dem Luftraum (4b) herzustellen, wobei ein geräuschabsorbierendes Material (6a1, 6b1) an mindestens einem von dem breiten und dem schmalen Bereich (6a, 6b) angebracht ist, um einen entsprechenden Teil des Einlassdurchgangs (6c) zu umgeben; wobei, wenn der Motor (4a) aktiviert ist, das Laufrad gedreht wird, um Luft aus dem Einlassdurchgang (6c) in den Luftraum (4b) zu ziehen und einen Strom einer Luftströmung durch den Auslass (4b2) des Luftraums auszugeben; und wobei ein Teil des Geräuschs, das aus der Bewegung von Luft in dem Luftraum (4b) resultiert, durch die Einlassgeräuschreduktionsstruktur (6) reduziert wird.

2. Luftgebläse nach Anspruch 1, ferner umfassend:
eine Auslassgeräuschreduktionsstruktur (14), beinhaltend ein Gehäuse mit mindestens einem großen Bereich (14a) und einem kleinen Bereich (14b), die einen Auslassdurchgang (14a3, 14b3) umgeben, wobei der große Bereich (14a) in Bezug auf den Luftraum (4b) so positioniert ist, dass ein Fluidkommunikationsweg zwischen einem Einlass (14i) der Auslassgeräuschreduktionsstruktur (14), dem Auslassdurchgang (14a3, 14b3) und dem Luftraum (4b) hergestellt wird, wobei ein geräuschabsorbierendes Material (14a1, 14b1) an mindestens einem von dem großen und dem kleinen Bereich (14a, 14b) angebracht ist, um einen entsprechenden Teil des Auslassdurchgangs (14a3, 14b3) zu umgeben;
wobei die Auslassgeräuschreduktionsstruktur (14) zusammen mit der Einlassgeräuschreduktionsstruktur (6) das Geräusch, das aus der Bewegung von Luft durch den Luftraum (4b) resultiert, im Wesentlichen reduziert.

3. Luftgebläse nach Anspruch 2, wobei eine Vielzahl von Löchern (6c1) entlang der rohrförmigen Wand ausgebildet ist, wobei die Löcher an der rohrförmigen Wand entlang des breiten Bereichs (6a) eine größere Abmessung als die Löcher an der rohrförmigen Wand entlang des schmalen Bereichs (6b) aufweisen;
wobei der Auslassdurchgang (14a3, 14b3) durch eine innere Umfangswand definiert ist, die sich entlang des großen und des kleinen Bereichs (14a1, 14b1) erstreckt, wobei eine Vielzahl von Löchern entlang der Umfangswand ausgebildet ist, wobei die Löcher an der Umfangswand entlang des großen Bereichs eine größere Abmessung als die Löcher an der Umfangswand entlang des kleinen Bereichs aufweisen.

4. Luftgebläse nach Anspruch 2, wobei jeder von dem breiten und dem schmalen Bereich (6a, 6b) der Einlassgeräuschreduktionsstruktur (6) mit dem geräuschabsorbierenden Material (6a1, 6b1) ausgestattet ist; und
wobei jeder von dem großen und dem kleinen Bereich (14a, 14b) der Auslassgeräuschreduktionsstruktur (14) mit dem Geräuschreduktionsmaterial (14a1, 14b1) ausgestattet ist;
wodurch das Geräusch, das aus dem Betrieb des Gebläses resultiert, als in einem Bereich von etwa 41 dBA bis 44,5 dBA gemessen wird.

5. Luftgebläse nach Anspruch 1, ferner umfassend mindestens ein Heizelement (12h1, 12h2), das in Bezug auf den Auslass (4b2) des Luftraums (4b) so positioniert ist, dass es die Luftströmung, die aus dem Auslass des Luftraums ausgegeben wird, erwärmt.

6. Luftgebläse nach Anspruch 2, ferner umfassend einen Heizluftraum (12), wobei mindestens ein Heizelement (12h1, 12h2) positioniert ist, wobei der Heizluftraum (12) zwischen dem Luftraum (4b) und der Auslassgeräuschreduktionsstruktur (14) angeordnet ist, wobei der Heizluftraum (12) einen Einlass (12a) zum Aufnehmen der aus dem Luftraum (4b) ausgegebenen Luft und einen Auslass (12d) zum Ausgeben der Luft, die durch das Heizelement (12h1, 12h2) erwärmt wurde, an den Einlass (14i) der Auslassgeräuschreduktionsstruktur (14) aufweist, sodass erwärmte Luft aus dem Auslass (20) des Auslassdurchgangs (14a3, 14b3) ausgegeben wird, und
wobei die Auslassgeräuschreduktionsstruktur (14) eine Auslassverlängerung (20) beinhaltet, die dazu ausgelegt ist, mit einem Ende eines Luftschlauchs (44) verbunden zu werden, dessen anderes Ende mit einer konvektiven Decke verbindbar ist, sodass die Decke durch die erwärmte Luft, die aus der Auslassgeräuschreduktionsstruktur (14) ausgegeben wird, aufgeblasen werden kann.

7. Luftgebläse nach Anspruch 1, wobei der breite Bereich (6a) durch eine breite Außenumfangswand definiert ist und der schmale Bereich (6b) durch eine schmale Außenumfangswand definiert ist, die einen Durchmesser aufweist, der kleiner als der Durchmesser der breiten Außenumfangswand ist, wobei ein Umfangsflansch ausgebildet ist, wo der breite Bereich (6a) auf den schmalen Bereich (6b) trifft, wobei das Luftgebläse ferner Folgendes umfasst:
einen Luftfilter (10), der entfernbar an dem Umfangsflansch befestigt ist, wobei der Filter eine umlaufende luftdurchlässige Barriere beinhaltet, die einen Hohlraum (10c) definiert, der den schmalen Bereich (6b) umgibt;
wobei Luft in den Hohlraum (10c) zu dem Einlassdurchgang (6c) gezogen wird, wenn das Luftgebläse in Betrieb ist.

8. Luftgebläse nach Anspruch 4, wobei der Auslassdurchgang (14a3, 14b3) durch eine Innenumfangswand ausgebildet ist, die sich entlang des großen und des kleinen Bereichs (14a, 14b) erstreckt; und
wobei die Innenumfangswand und die rohrförmige Wand von jedem von dem Einlass- und dem Auslassdurchgang (6c; 14a3, 14b3) durch das geräuschabsorbierende Material (6a1, 6b1; 14a1, 14b1) umgeben ist.

9. Luftgebläse nach Anspruch 1, wobei der breite Bereich (6b) des Gehäuses mit dem geräuschabsorbierenden Material (6a1, 6b1; 14a1, 14b1) ausgestattet ist und die schmalen Bereiche eine Helmholtz-Kammer sind.

10. Luftgebläse nach Anspruch 2, wobei der Auslassdurchgang (14a3, 14b3) mindestens innerhalb des großen und des kleinen Bereichs (14a, 14b) des Gehäuses der Auslassgeräuschreduktionsstruktur (14) ausgebildet ist;
wobei einer von dem großen und dem kleinen Bereich (14a, 14b) des Gehäuses mit dem geräuschabsorbierenden Material (14a1, 14b1) ausgestattet ist und ein anderer von dem großen und dem kleinen Bereich eine Helmholtz-Kammer ist;
wobei der geräuschabsorbierend ausgestattete Bereich niederfrequente Geräusche dämpft und die Helmholtz-Kammer hochfrequente Geräusche dämpft.

11. Luftgebläse nach Anspruch 1, ferner umfassend einen Luftauslass (20), der dazu ausgelegt ist, an unterschiedliche Typen von Luftschläuchen gekoppelt zu werden, die jeweils an ihrem patientenseitigen Ende mindestens einen Sensor (s2, s2') bereitstellen, wobei mindestens ein anderer Sensor (s1) an dem Luftauslass (20) des Luftgebläses bereitgestellt ist, wobei der eine Sensor (s2, s2') die Temperatur der erwärmten Luft an dem patientenseitigen Ende eines Luftschlauchs (44) misst, der an den Luftauslass (20) gekoppelt ist, und der andere Sensor (s1) die Temperatur der erwärmten Luft an dem Luftauslass misst, eine Schaltung, die die Werte des einen und des anderen Sensors mit Referenzwerten nutzt, um eine Referenztabelle mit gestuften Werten zu erstellen, die jedem Typ der unterschiedlichen Typen von Luftschläuchen entsprechen, die ausgelegt sind, um an den Luftauslass (20) gekoppelt zu werden, wobei die Schaltung, wenn ein bestimmter Luftschlauch an den Luftauslass gekoppelt ist, den Motor (4a) dazu veranlasst, das Laufrad mit einer Rate gemäß einem Stufenwert zu drehen, der dem Typ des Luftschlauchs entspricht, der der bestimmte Luftschlauch ist.

12. Luftgebläse nach Anspruch 1, wobei die Einlassgeräuschstruktur (6) Folgendes umfasst
einen Einlassschalldämpfer (6), der an den Luftraum (4b) gekoppelt ist, wobei der Einlassschalldämpfer (6) das Gehäuse (6a, 6b) mit einer Außenwand und einem breiten Bereich (6a) und einem schmalen Bereich (6b) beinhaltet, wobei der Einlassdurchgang (6c) in Fluidkommunikation mit dem Einlass (4b1) des Luftraums (4b) steht, wobei das schallabsorbierende Material (6a1, 6b1) zwischen der Außenwand des breiten Bereichs (6a) des Gehäuses (6a, 6b) und der rohrförmigen Wand des Gehäuses, die den Einlassdurchgang (6c) definiert, angebracht ist; wobei das Luftgebläse ferner Folgendes umfasst
einen Auslassschalldämpfer (14), der ein zweites Gehäuse (14a, 14b) mit einer Außenwand und einem Auslassdurchgang (14a3, 14b3) beinhaltet, wobei der Auslassschalldämpfer (14) in Bezug auf den Luftraum so positioniert ist, dass der Auslassdurchgang (14a3, 14b3) in Fluidkommunikation mit dem Auslass (4b2) des Luftraums steht, wobei ein schallabsorbierendes Material (14a1, 14b1) zwischen der Außenwand und einer Innenumfangswand des zweiten Gehäuses, die den Auslassdurchgang (14a3, 14b3) definiert, angebracht ist; und
einen Luftfilter, der an den Einlassschalldämpfer gekoppelt ist, um Luft zu filtern, die in den Einlassdurchgang (6c) gesaugt wird, wenn die Motorbaugruppe in Betrieb ist;
wobei, wenn der Motor (4a) in Betrieb ist, die in den Einlass des Einlassdurchgangs (6c) gesaugte Luft zu dem Luftraum strömt und von dort als ein Strom einer Luftströmung zu dem Auslassdurchgang (14a3, 14b3) ausgegeben wird; und
wobei Schall, der mindestens aus Luftturbulenzen aufgrund von Bewegung der Luft in dem Luftraum während eines Betriebs der Motorbaugruppe resultiert, durch den Einlass- und den Auslassschalldämpfer reduziert wird.

13. Luftgebläse nach Anspruch 2, wobei die Einlassgeräuschreduktionsstruktur (6) ein Einlassschalldämpfer ist, der den breiten Bereich (6a) und den schmalen Bereich (6b) beinhaltet; und
wobei die Auslassgeräuschreduktionsstruktur (14) ein Auslassschalldämpfer ist, der den großen Bereich (14a), der als ein Niederfrequenzgeräuschdämpfungsbereich wirkt, um Geräusche der niedrigen Frequenzen zu dämpfen, und den kleinen Bereich (14b), der als ein Hochfrequenzdämpfungsbereich wirkt, um Geräusche der höheren Frequenzen zu dämpfen, beinhaltet;
wobei, wenn der Motor aktiviert ist, um das Laufrad in dem Luftraum zu drehen, Luft in den Einlassschalldämpfer gesaugt wird und ein Strom einer Luftströmung aus dem Auslassschalldämpfer ausgegeben wird; und
wobei der Einlass- und der Auslassschalldämpfer wirken, um Geräusche zu reduzieren, die aus dem Betrieb des Luftgebläses resultieren.

14. Luftgebläse nach Anspruch 13, ferner umfassend:
einen Luftfilter (10), der über dem Einlass (6f) des Einlassschalldämpfers (6) angebracht ist, um die in den Einlassschalldämpfer gesaugte Luft zu filtern;
eine Ummantelung (32) zum Unterbringen mindestens der Motorbaugruppe (4), des Einlass- und des Auslassschalldämpfers (6, 14) und des Heizluftraums (12);
wobei das Geräusch, das aus dem Betrieb des Luftgebläses resultiert, als zwischen etwa 42 dBA bis 43 dBA gemessen wird.

15. Luftgebläse nach Anspruch 13, wobei mindestens einer von dem Niederfrequenz- und dem Hochfrequenzdämpfungsbereich (14a, 15b) in dem Auslassschalldämpfer (6, 14) mit einem geräuschabsorbierenden Material (6a1, 6b1; 14a1, 14b1) ausgestattet ist.

## Revendications

1. Soufflante d'air (2) comportant un plénum (4b) dans lequel une roue est positionnée de manière mobile, le plénum possédant une entrée (4b1) et une sortie (4b2), un moteur (4a) fonctionnant en coopération avec la roue pour faire tourner la roue et une structure de réduction de bruit d'entrée (6) positionnée par rapport au plénum (4b) comprenant un boîtier présentant au moins une section large (6a) et une section étroite (6b), la section large (6a) présentant un diamètre plus grand que la section étroite (6b), la section large (6a) est conçue pour atténuer les bruits à basse fréquence tandis que la section étroite (6b) est conçue pour atténuer les bruits à fréquences plus élevées à travers la large bande de fréquences de bruit, les sections large et étroite (6a, 6b) entourant un alésage coaxial central commun (6c) défini par une paroi tubulaire qui s'étend à travers les sections large et étroite de manière à réaliser un passage traversant (6c) possédant une entrée (6f) ouverte sur l'environnement, la section large (6a) étant positionnée adjacente au plénum (4b) de sorte qu'une sortie (6g) du passage traversant d'entrée (6c) soit en communication fluidique avec l'entrée (4b1) du plénum (4b) afin d'établir un trajet de communication fluidique entre le passage traversant d'entrée (6c) et le plénum (4b), un matériau d'absorption de bruit (6a1, 6b1) étant installé sur au moins l'une des sections large et étroite (6a, 6b) pour entourer une partie correspondante du passage de traversant d'entrée (6c) ; lorsque le moteur (4a) est actionné, ladite roue étant tournée pour aspirer de l'air dans le plénum (4b) à partir du passage traversant d'entrée (6c) et pour délivrer un flux d'écoulement d'air à travers la sortie (4b2) du plénum ; et une partie du bruit résultant du déplacement de l'air dans le plénum (4b) étant réduite par la structure de réduction de bruit d'entrée (6).

2. Soufflante d'air selon la revendication 1, comprenant en outre :
une structure de réduction de bruit de sortie (14) comprenant un boîtier présentant au moins une grande section (14a) et une petite section (14b) entourant un passage traversant de sortie (14a3, 14b3), la grande section (14a) étant positionnée par rapport au plénum (4b) de sorte qu'un passage de communication fluidique soit établi entre une entrée (14i) de la structure de réduction de bruit de sortie (14), le passage traversant de sortie (14a3, 14b3) et le plénum (4b), un matériau d'absorption de bruit (14a1, 14b1) étant installé sur au moins l'une des grande et petite section (14a, 14b) afin d'entourer une partie correspondante du passage traversant de sortie (14a3, 14b3) ;
ladite structure de réduction de bruit de sortie (14) ainsi que ladite structure de réduction de bruit d'entrée (6) réduisant sensiblement le bruit résultant du déplacement de l'air à travers le plénum (4b).

3. Soufflante d'air selon la revendication 2, une pluralité de trous (6c1) étant formés le long de la paroi tubulaire, lesdits trous au niveau de la paroi tubulaire le long de la section large (6a) présentant une dimension plus grande que les trous au niveau de la paroi tubulaire le long de la section étroite (6b) ;
ledit passage traversant de sortie (14a3, 14b3) étant défini par une paroi circonférentielle interne qui s'étend le long des grande et petite sections (14a1, 14b1), une pluralité de trous formés le long de la paroi circonférentielle, les trous au niveau de la paroi circonférentielle le long de la grande section présentant une dimension plus grande que les trous au niveau de la paroi circonférentielle le long de la petite section.

4. Soufflante d'air selon la revendication 2, chacune des sections large et étroite (6a, 6b) de la structure de réduction de bruit d'entrée (6) étant équipée du matériau d'absorption de bruit (6a1, 6b1) ; et
chacune desdites grande et petite sections (14a, 14b) de la structure de réduction de bruit de sortie (14) étant équipée du matériau de réduction de bruit (14a1, 14b1) ;
moyennant quoi le bruit résultant du fonctionnement de la soufflante est mesuré comme se trouvant dans une plage d'approximativement 41 dBA à 44,5 dBA.

5. Soufflante d'air selon la revendication 1, comprenant en outre au moins un élément chauffant (12h1, 12h2) positionné par rapport à la sortie (4b2) du plénum (4b) de manière à chauffer le flux d'air sortant de la sortie du plénum.

6. Soufflante d'air selon la revendication 2, comprenant en outre un plénum chauffant (12) dans lequel au moins un élément chauffant (12h1, 12h2) est positionné, le plénum chauffant (12) étant intercalé entre le plénum (4b) et la structure de réduction de bruit de sortie (14), ledit plénum chauffant (12) possédant une entrée (12a) destinée à recevoir la sortie d'air du plénum (4b) et une sortie (12d) destinée à délivrer l'air ayant été chauffé par l'élément chauffant (12h1, 12h2) vers l'entrée (14i) de la structure de réduction de bruit de sortie (14) de sorte que l'air chauffé soit délivré depuis la sortie (20) du passage traversant de sortie (14a3, 14b3) et
ladite structure de réduction de bruit de sortie (14) comprenant une extension de sortie (20) adaptée à se raccorder à une extrémité d'un tuyau d'air (44), dont l'autre extrémité peut être raccordée à une couverture à convection de sorte que ladite couverture puisse être gonflée par la sortie d'air chauffé provenant de ladite structure de réduction de bruit de sortie (14).

7. Soufflante d'air selon la revendication 1, ladite section large (6a) étant définie par une paroi circonférentielle externe large et ladite section étroite (6b) étant définie par une paroi circonférentielle externe étroite qui présente un diamètre inférieur au diamètre de la paroi circonférentielle externe large, une bride circonférentielle étant formée à l'endroit où la section large (6a) rejoint la section étroite (6b), la soufflante d'air comprenant en outre :
un filtre à air (10) fixé de manière amovible à la bride circonférentielle, le filtre comprenant une barrière de passage d'air circonférentielle définissant une cavité (10c) entourant la section étroite (6b) ;
ledit air étant aspiré dans la cavité (10c) vers le passage d'entrée (6c) lorsque la soufflante d'air est en fonctionnement.

8. Soufflante d'air selon la revendication 4, ledit passage traversant de sortie (14a3, 14b3) étant formé d'une paroi circonférentielle interne qui s'étend le long des grande et petite sections (14a, 14b) ; et
ladite paroi circonférentielle interne et ladite paroi tubulaire de chacun des passages traversants d'entrée et de sortie (6c ; 14a3, 14b3) étant circonscrites par le matériau d'absorption de bruit (6a1, 6b1 ; 14a1, 14b1).

9. Soufflante d'air selon la revendication 1, ladite section large (6b) du boîtier étant équipée du matériau d'absorption de bruit (6a1, 6b1 ; 14a1, 14b1) et lesdites sections étroites étant une chambre de Helmholtz.

10. Soufflante d'air selon la revendication 2, ledit passage traversant de sortie (14a3, 14b3) étant formé au moins à l'intérieur des grande et petite sections (14a, 14b) du boîtier de la structure de réduction de bruit de sortie (14) ;
l'une desdites grande et petite sections (14a, 14b) du boîtier étant équipée du matériau d'absorption de bruit (14a1, 14b1) et l'autre desdites grande et petite sections étant une chambre de Helmholtz ;
ladite section ajustée d'absorption de bruit atténuant le bruit à basse fréquence et ladite chambre de Helmholtz atténuant le bruit à haute fréquence.

11. Soufflante d'air selon la revendication 1, comprenant en outre une sortie d'air (20) adaptée au couplage à différents types de tuyaux d'air, chacun étant pourvu au niveau de son extrémité patient d'au moins un capteur (s2, s2'), d'au moins un autre capteur (s1) pourvu au niveau de la sortie d'air (20) de la soufflante, ledit capteur (s2, s2') mesurant la température de l'air chauffé au niveau de l'extrémité patient d'un tuyau d'air (44) couplé à la sortie d'air (20) et à l'autre capteur (s1) mesurant la température de l'air chauffé au niveau de la sortie d'air, un circuit qui utilise les valeurs de l'un et de l'autre capteur avec des valeurs de référence afin d'établir une table de référence de valeurs échelonnées qui correspondent à chaque type des différents types de tuyaux d'air adaptés au couplage à la sortie d'air (20), lorsqu'un tuyau d'air donné est couplé à la sortie d'air, ledit circuit amenant le moteur (4a) à faire tourner la roue à une vitesse conformément à une valeur d'échelon qui correspond au type de tuyau d'air qui est ledit tuyau d'air donné.

12. Soufflante d'air selon la revendication 1, ladite structure de bruit d'entrée (6) comprenant
un silencieux d'entrée (6) couplé au plénum (4b), le silencieux d'entrée (6) comprenant le boîtier (6a, 6b) présentant une paroi externe et une section large (6a) et une section étroite (6b), le passage traversant d'entrée (6c) étant en communication fluidique avec l'entrée (4b1) du plénum (4b), le matériau d'absorption de son (6a1, 6b1) étant installé entre la paroi externe de la section large (6a) du boîtier (6a, 6b) et la paroi tubulaire du boîtier qui définit le passage traversant d'entrée (6c) ; la soufflante d'air comprenant en outre
un silencieux de sortie (14) comprenant un second boîtier (14a, 14b) présentant une paroi externe et un passage traversant de sortie (14a3, 14b3), le silencieux de sortie (14) étant positionné par rapport au plénum, le passage traversant de sortie (14a3, 14b3) étant en communication fluidique avec la sortie (4b2) du plénum, un matériau d'absorption de son (14a1, 14b1) étant installé entre la paroi externe et une paroi circonférentielle interne du second boîtier qui définit le passage traversant de sortie (14a3, 14b3) ; et
un filtre à air couplé au silencieux d'entrée de manière à filtrer l'air aspiré dans le passage d'entrée (6c) lorsque l'ensemble moteur est en fonctionnement ;
lorsque ledit moteur (4a) est en fonctionnement, ledit air aspiré dans l'entrée du passage traversant d'entrée (6c) passant vers le plénum et sortant de celui-ci vers le passage traversant de sortie (14a3, 14b3) sous la forme d'un flux d'écoulement d'air ; et
ledit son résultant au moins de la turbulence de l'air due au déplacement de l'air dans le plénum pendant le fonctionnement de l'ensemble moteur étant réduit par les silencieux d'entrée et de sortie.

13. Soufflante d'air selon la revendication 2, ladite structure de réduction de bruit d'entrée (6) étant un silencieux d'entrée comprenant la section large (6a) et la section étroite (6b) ; et
ladite structure de réduction de bruit de sortie (14) étant un silencieux de sortie comprenant la grande section (14a) agissant comme une section d'atténuation de bruit à basse fréquence pour atténuer le bruit des basses fréquences et la petite section (14b) agissant comme une section d'atténuation des hautes fréquences pour atténuer le bruit des fréquences plus élevées ;
lorsque le moteur est activé pour faire tourner la roue dans le plénum, de l'air est aspiré dans le silencieux d'entrée et un flux d'écoulement d'air est délivré par le silencieux de sortie ; et
les silencieux d'entrée et de sortie agissent pour réduire le bruit résultant du fonctionnement de la soufflante d'air.

14. Soufflante d'air selon la revendication 13, comprenant en outre :
un filtre à air (10) monté sur l'entrée (6f) du silencieux d'entrée (6) pour filtrer l'air aspiré dans le silencieux d'entrée ;
un carter (32) pour loger au moins l'ensemble moteur (4), les silencieux d'entrée et de sortie (6, 14) et le plénum chauffant (12) ;
ledit bruit résultant du fonctionnement de la soufflante étant mesuré entre approximativement 42 dBA à 43 dBA.

15. Soufflante d'air selon la revendication 13, au moins l'une des sections d'atténuation de basses fréquences et de hautes fréquences (14a, 15b) dans le silencieux de sortie (6, 14) étant équipée d'un matériau d'absorption de bruit (6a1, 6b1 ; 14a1, 14b1).
